# FASCICULE DE BREVET EUROPEEN

(11) **EP 2 450 083 B1**
(45) Date de publication et mention de la délivrance du brevet: **07.08.2013**
(21) Numéro de dépôt: 10189893.0
(22) Date de dépôt: 03.11.2010
(51) Int. Cl.: A61N 5/10, A61B 6/08, A61B 6/00

(54) **Objet-test pour le contrôle qualité d'un appareil de traitement par radiothérapie et procédés de fabrication et d'utilisation dudit objet-test**
Testobjekt für die Qualitätskontrolle eines Geräts zur Strahlentherapie-Behandlung, sowie Herstellungs- und Anwendungsverfahren dieses Testobjekts
Test object for quality control of a radiotherapy treatment device and methods for manufacturing and using said test object

(43) Date de publication de la demande: 09.05.2012
(73) Titulaire: Qualiformed, 85000 La Roche-sur-Yon (FR)
(72) Inventeur: Beaumont, Stéphane, 85430, NIEUL LE DOLENT (FR); Villing, Margit, 85430, NIEUL LE DOLENT (FR); Barbotteau, Yves, 13010, MARSEILLE (FR); Boucenna, Rachid, 74800, LA ROCHE SUR FORON (FR)
(74) Mandataire: Chaillot, Geneviève

(56) Documents cités:
- DE-A1- 19 907 065
- FR-A1- 2 945 955
- US-A- 5 281 232
- US-A1- 2006 002 519
- US-A1- 2008 049 412

## Description

La présente invention porte sur un objet-test pour le contrôle qualité d'appareils de traitement par radiothérapie, sur un procédé de fabrication de l'objet-test et sur des procédés d'utilisation de cet objet-test, consistant en un procédé de vérification de la coïncidence, de l'orthogonalité, et de la position dans l'espace des moyens de repérage des trois axes de rotation théoriques d'un appareil de traitement par radiothérapie et en un procédé de recherche de l'isocentre d'un appareil de traitement par radiothérapie utilisant l'objet-test, ces derniers étant les deux aspects du contrôle-qualité.

Le traitement par radiothérapie, dans le cadre du traitement du cancer, doit être effectué de telle sorte que les rayons soient ciblés sur la tumeur et épargnent au maximum les tissus sains autour de la tumeur traitée.

Pour garantir ce ciblage des rayons sur la tumeur, plusieurs tests de contrôle qualité des appareils de traitement ont été mis au point, lesquels sont effectués sur l'appareil de traitement avant le traitement par radiothérapie d'un patient.

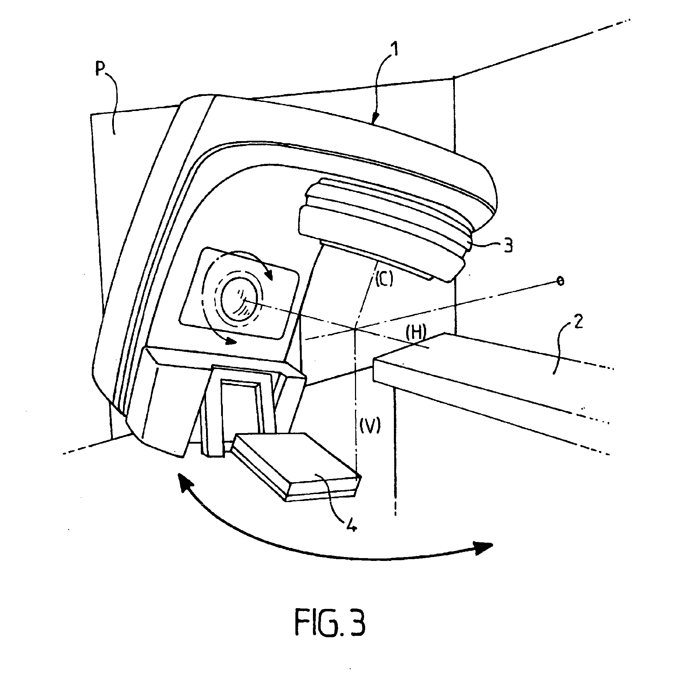

Un appareil de traitement par radiothérapie classique, représenté par exemple sur la Figure 3, comprend un statif, portant à une extrémité une tête d'irradiation qui se termine par un collimateur qui permet de délimiter le faisceau de rayonnement (ou faisceau d'irradiation) et à l'autre extrémité un imageur appelé imageur portal qui permet de faire des radiographies numériques d'un objet placé entre le collimateur et l'imageur en général sur une table de traitement également appelée support patient.

L'appareil de traitement comporte trois axes de rotation, représentés sur la Figure 3 : l'axe horizontal de rotation du statif, permettant de faire tourner la tête d'irradiation autour du patient traité, l'axe de rotation du collimateur, qui est un axe qui passe par le centre du collimateur, et qui est perpendiculaire à l'axe de rotation horizontal du statif, cet axe étant confondu avec l'axe vertical passant par le centre du collimateur lorsque le statif a un angle de rotation nul, et l'axe vertical de rotation du support patient, qui est un axe qui passe par le centre du collimateur, lorsque le statif a un angle de rotation nul.

Le point de concours de ces trois axes est appelé l'isocentre.

La position et la « taille » de cet isocentre sont fondamentales à connaître car c'est sur ce point dans l'espace tridimensionnel de la salle de traitement que le centre de la tumeur à traiter sera positionné afin de pouvoir l'irradier au moyen de multiples faisceaux concentriques. Ce point d'isocentre est matérialisé dans les salles de traitement par cinq nappes laser orthogonales, deux frontales, une sagittale et deux transversales. Ces nappes vont permettre d'aligner trois points de repère (un antérieur et deux latéraux) matérialisés sur la peau du patient (tatouage) ou sur la surface d'un système de contention utilisé pour positionner de manière très précise le patient, lors de la phase de préparation et planification du traitement.

Les deux nappes transversales sont idéalement dans un plan vertical, orthogonal à la direction longitudinale du support-patient lorsque celui-ci a un angle de rotation nul, les deux nappes frontales sont idéalement dans un plan horizontal, et la nappe sagittale est dans un plan vertical, orthogonal au plan des nappes transversales.

Pour s'assurer de la précision géométrique de l'irradiation, il est primordial de vérifier que l'isocentre théorique matérialisé par l'intersection des nappes laser de repérage est bien aligné avec l'isocentre réel de l'appareil de traitement, qui correspond à l'intersection des trois axes de rotation réels de l'appareil de traitement par radiothérapie. Tout désalignement entre l'isocentre réel et l'isocentre théorique se traduirait d'une part, par une irradiation incomplète de la tumeur, ce qui peut induire une récidive de la maladie, et, d'autre part, par une irradiation des tissus sains avoisinant la tumeur, laquelle irradiation est susceptible de produire des complications graves.

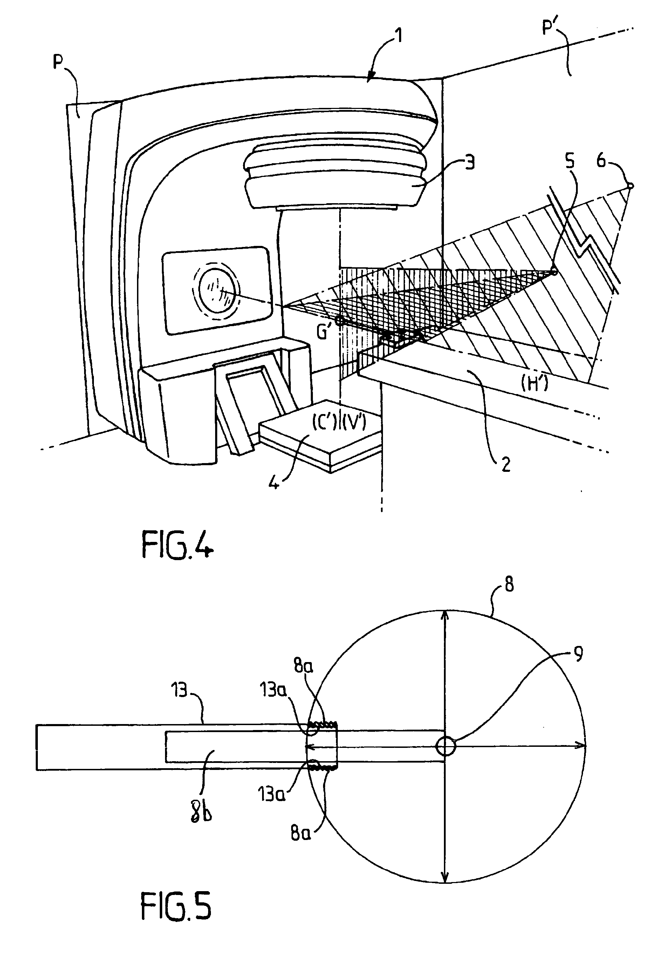

Le test de Winston-Lutz (W&L) permet de vérifier la coïncidence de l'isocentre théorique et de l'isocentre réel. Il consiste à aligner une bille radio-opaque, donc de densité électronique importante, notamment de l'ordre de celle d'un métal, sphérique (le plus souvent en acier) sur l'isocentre théorique de l'appareil (intersection des nappes laser de repérage) et à réaliser de multiples radiographies de cet objet avec les rayons X issus de faisceaux de traitement. En faisant de telles radiographies pour différents angles de rotation du statif, on détermine la position et la « taille » de l'axe de rotation du statif. En faisant ces radiographies pour différents angles de rotation du support patient, on détermine la position et la « taille » de l'axe de rotation du support patient. Enfin en faisant ces radiographies pour différents angles de rotation du collimateur, on détermine la position et la « taille » de l'axe de rotation du collimateur. Il est à noter que pendant le test de W&L, on effectue une série de radiographies autour d'un seul axe de rotation à la fois, les autres angles de rotation autour des deux autres axes étant fixés à 0°. L'angle de 0° pour le statif correspond à la position verticale de celui-ci (comme représenté sur la Figure 4), l'angle de 0° pour le support patient correspond à la position du support patient dans laquelle la direction longitudinale du support patient est alignée avec l'axe de rotation du statif, et l'angle de 0° pour le collimateur correspond à un angle prédéterminé dans le collimateur. Ce test de Winston-Lutz est décrit sur la page internet http://www.wienkav.at/kav/kfj/91033454/physik/aS500/aS500₋s phere.htm

Le terme « taille » utilisé pour définir une caractéristique d'un axe de rotation, tel qu'utilisé ci-dessus pour les axes de rotation réels, correspond au diamètre moyen de l'axe de rotation considéré, qui dans le cas des axes de rotation réels, ne correspond pas exactement à une ligne, mais est contenu dans un cylindre très mince.

Si un objet-test constitué d'une bille radio-opaque est parfaitement positionné sur l'isocentre réel de l'appareil, ce qui signifie que l'isocentre théorique (lasers de repérage) coïncide parfaitement avec l'isocentre réel de l'appareil de traitement, alors l'image de la bille sur les radiographies récurrentes est constante.

Sinon, l'image de la bille radio-opaque décrit un mouvement dont l'analyse permet de trouver les décalages à produire pour réaligner l'isocentre théorique sur l'isocentre réel de l'appareil de traitement.

La bille radio-opaque doit avoir des dimensions suffisamment faibles pour qu'elle puisse être contenue dans un faisceau d'irradiation de faible section (environ 50 mm) délimité par le collimateur. En effet les mouvements de la bille radio-opaque ne sont pas étudiés par rapport à une origine liée au détecteur de rayonnement, mais par rapport au centre du faisceau d'irradiation repéré sur le détecteur de rayonnement. Ceci permet en effet de s'affranchir d'un éventuel mouvement du détecteur de rayonnement pendant la rotation du statif tournant, mouvement qui serait interprété comme un défaut sur l'isocentre de l'appareil de traitement. Pour limiter également un éventuel mouvement avec la rotation du statif du système de limitation du faisceau contenu dans le collimateur, on a recours à des faisceaux de faible section. Il existe plusieurs objets-test pour réaliser le test de W&L. L'objet-test le plus utilisé est une bille radio-opaque de diamètre compris entre 2 ou 10 mm qu'il convient d'aligner sur les cinq nappes laser de repérage. Cette opération est difficile car, en général, la bille ne peut pas porter de repères d'alignement sur les nappes laser du fait de ses faibles dimensions. Même si elle était gravée, toujours en raison du fait de ses faibles dimensions, l'alignement des lasers n'est contrôlable que sur une surface réduite de la bille, ce qui conduit à une imprécision de placement de la bille de l'ordre des décalages entre les isocentres réel et théorique que l'on souhaite mesurer et corriger. Une solution consisterait à réduire encore les dimensions de la bille pour préciser l'alignement des lasers, mais dans ce cas la bille de moins en moins opaque serait difficilement repérée dans les radiographies récurrentes.

D'autres objets-test reposent sur le principe qui consiste à enfermer la bille dans un parallélépipède en plastique de faibles dimensions (20 cm³) comportant un réticule gravé sur trois des six faces, la face antérieure et les deux faces latérales. Cette fois, l'alignement de la bille est facilité mais la géométrie parallélépipédique rend les radiographies récurrentes disparates du fait des variations de l'image de la projection du parallélépipède en fonction de l'angle de projection. Cet inconvénient empêche une détection optimale du centre de la bille radio-opaque, notamment avec l'utilisation de logiciel d'analyse automatique des radiographies. Par ailleurs la géométrie parallélépipédique empêche de vérifier la coïncidence des nappes lasers et les faibles dimensions du parallélépipède englobant limitent la précision du contrôle préalable de l'orthogonalité et de la position dans l'espace (horizontalité et verticalité) des nappes lasers.

Les objets test utilisés actuellement présentent donc deux inconvénients majeurs : d'une part l'imprécision du placement de la bille radio-opaque à l'intersection des cinq nappes laser, c'est-à-dire sur l'isocentre théorique, et d'autre part l'impossibilité de vérifier, préalablement ou de manière complémentaire au test de W&L, l'orthogonalité la coïncidence, et la position dans l'espace des nappes laser donc des trois axes théoriques.

En effet, deux des trois axes théoriques (l'axe de rotation du collimateur et du support-patient) doivent être verticaux, le dernier axe théorique (axe de rotation du statif) devant être horizontal).

Le brevet américain US 5281232 décrit un objet-test, constitué d'une bille et d'une sphère entourant la bille, le diamètre de la sphère étant inférieur au faisceau d'irradiation.

La présente invention surmonte les inconvénients des objets-test de l'état antérieur de la technique, et porte sur un objet-test pour un test de contrôle qualité d'un appareil de traitement par radiothérapie, ledit appareil de traitement par radiothérapie comprenant :
- un support patient pour un placement d'un patient en vue d'un traitement par radiothérapie du patient, ledit support patient étant mobile en rotation autour d'un axe vertical (V) ;
- un statif portant à une extrémité la tête d'irradiation qui se termine par un collimateur permettant de délimiter un faisceau de rayonnement et à l'autre extrémité des moyens de détection de rayonnement, le statif étant mobile en rotation autour d'un axe de rotation horizontal (H), le collimateur étant mobile en rotation autour d'un axe (C) parallèle à la direction du faisceau émis et passant par le centre du collimateur,
   des moyens de repérage externes à et/ou portés par l'appareil de traitement par radiothérapie étant prévus pour représenter visuellement les positions théoriques (V', H', C') des trois axes (V, H, C), le test de contrôle qualité comportant l'opération consistant à faire des images successives d'une bille sphérique faite d'un matériau ayant une densité électronique d1, et placée au point de concours des axes théoriques (V', H', C'),
   caractérisé par le fait que la bille sphérique de densité électronique d1 est disposée au centre d'une sphère faite d'un matériau ayant une densité électronique d2 afin de constituer avec elle l'objet-test, la section diamétrale de ladite bille étant inférieure à la section dudit faisceau de rayonnement et la section diamétrale de ladite sphère étant supérieure à la section dudit faisceau de rayonnement, le rapport de la densité électronique d1 sur la densité électronique d2 étant supérieur ou égal à 1,1, et ladite sphère portant sur sa surface externe des moyens d'alignement visuel avec les moyens de repérage permettant un positionnement de l'objet-test, de telle sorte que ladite bille sphérique vient à être correctement placée au point de concours des trois axes (V', H', C'), ladite sphère comportant des moyens permettant de la rendre solidaire de moyens de mise en place de l'objet-test sur l'appareil de traitement par radiothérapie.

En particulier, l'objet-test de l'invention est défini dans la revendication 1. Les caractéristiques particulières sont contenues dans les revendications dépendantes.

Ce test de contrôle qualité est le test de Winston-Lutz décrit plus haut.

La section diamétrale d'une bille ou d'une sphère est définie comme la section de cette bille ou de cette sphère suivant un de ses diamètres.

La densité électronique d'un matériau est définie comme étant le nombre d'électrons contenus par centimètrecube. Elle peut s'exprimer relativement à l'eau. Elle se calcule comme le produit de : Masse volumique du matériau (g/cm³) ^{*} Nombre d'Avogadro (6,0228 10²³ atomes/mole) ^{*} Z (Numéro atomique du matériau) / A (masse atomique du matériau).

Cette densité électronique est souvent exprimée relativement à celle de l'eau (densité électronique relative).

Dans ce qui suit, la densité électronique relative d'un matériau signifiera la densité électronique de ce matériau par rapport à celle de l'eau, ou densité électronique du matériau divisée par la densité électronique de l'eau (-3,34 10²³ électrons/cm3)

Les rapports de densité électronique entre le matériau de densité électronique d1 et le matériau de densité électronique d2 (d1/d2) sont avantageusement compris entre 2 et 20, de préférence 12.

Un rapport de densité électronique entre le matériau de densité électronique d1 et le matériau de densité électronique d2 de 12 donne des résultats optimaux pour le test de contrôle-qualité.

Les mesures de la position de l'image de la bille de densité électronique d1 sur le détecteur de rayonnement sont faites par rapport à l'image du faisceau de rayonnement sur le détecteur, la position de la bille étant détectée par rapport à l'image des bords du faisceau sur le détecteur.

La densité électronique de la bille (d1) est plus élevée que la densité électronique de la sphère (d2), de telle sorte que l'on a un contraste de densité électronique entre la bille et la sphère. La radio-opacité d'un matériau étant une fonction croissante de la densité électronique de ce matériau, la bille (de densité électronique plus élevée) est plus radio-opaque que la sphère (de densité électronique plus faible).Donc, la sphère de densité électronique d2 (faible par rapport à la densité électronique forte d1 de la bille) présentant une symétrie de rotation, si la section du faisceau de rayonnement est inférieure au diamètre de la sphère, la rotation du faisceau autour de la sphère sera invisible sur le détecteur, et les méthodes de détection des bords du faisceau et du centre de la bille seront strictement les mêmes, quel que soit l'angle d'incidence et l'orientation du faisceau. Par ailleurs, la section diamétrale de la sphère de densité électronique faible étant supérieure à la section du faisceau, les bords du faisceau seront facilement repérés sur le détecteur car ce repérage ne sera pas perturbé par l'image des limites de la sphère qui seront ainsi invisibles.

De même, la section du faisceau étant supérieure à la section diamétrale de la bille, la bille de densité électronique d1 sera facilement repérée sur le détecteur sans venir perturber la détection des bords du faisceau.

La section diamétrale de la sphère doit être supérieure à la section du faisceau, c'est-à-dire que le diamètre de la sphère doit être supérieur au diamètre du faisceau si le faisceau est à section circulaire, ou à la diagonale du faisceau si le faisceau est à section carrée. De préférence, la sphère aura un diamètre supérieur de 20 mm à la section du faisceau.

La section diamétrale de la bille doit être inférieure à la section du faisceau, c'est-à-dire que le diamètre de la bille doit être inférieur au diamètre du faisceau si le faisceau est à section circulaire, ou à la diagonale du faisceau si le faisceau est à section carrée. De préférence, le rapport entre la section diamétrale de la bille et la section du faisceau sera 1/10.

Avantageusement, le matériau de densité électronique d2 pourra avoir une densité électronique proche de celle de tissus vivants humains, et notamment une densité électronique relative (par rapport à l'eau) de l'ordre de 1,15.

Ainsi, avec cet objet-test, on garde le diamètre de la bille de matériau de densité électronique d1 suffisamment petit pour qu'elle puisse être contenue dans un faisceau d'irradiation de faible section donc peu soumis des variations de ses dimensions en fonction de la rotation du statif, les moyens de positionnement-alignement étant portés par une sphère en matériau de densité électronique d2 de diamètre suffisant d'une part pour porter des moyens qui permettent à un opérateur de placer avec précision l'objet-test dans l'appareil de traitement à l'oeil nu, et, d'autre part, contenir en totalité la faible section du faisceau d'irradiation utilisé pour les tests.

La sphère en matériau de densité électronique d2 peut notamment avoir de grandes dimensions (environ 500 cm³) . Les moyens de repérage peuvent être des nappes laser projetées par des projecteurs laser, par exemple de couleur rouge ou de couleur verte, placés dans la salle de traitement.

Les moyens d'alignement-positionnement de la sphère en matériau de densité électronique d2 portés par la sphère permettent donc un positionnement précis de la sphère par rapport aux moyens de repérage constitués par trois nappes laser de repérage, étant donné que les dimensions de la sphère en matériau de densité électronique d2, plus importantes que les dimensions de la bille en matériau de densité électronique d1, permettent un placement visuel aisé et précis de l'objet-test par rapport aux nappes laser de repérage par l'opérateur effectuant le test de contrôle qualité, ce placement étant contrôlé sur une plus grande longueur (le diamètre de la sphère) que pour les objets test de l'état antérieur de la technique (diamètre de la bille).

Le matériau de densité électronique d2 à partir duquel est fabriquée la sphère peut être une matière plastique, en particulier une matière plastique transparente pour la lumière visible, notamment en poly(méthacrylate de méthyle) (PMMA). On peut ainsi s'assurer visuellement du bon positionnement de la bille en matériau de densité électronique d1 au centre de la sphère. Le matériau de densité électronique d2 de la sphère permet que celle-ci soit invisible sur les images de radiographie prises pendant le test, seule la bille en matériau de densité électronique d1 au centre étant visible sur ces images.

Les moyens d'alignement-positionnement de la sphère sont constitués par trois lignes équatoriales visibles, orthogonales deux à deux, formées sur la surface de la sphère en matériau de densité électronique d2, lesdites lignes peuvent être matérialisées par un trait peint ou imprimé, ou par une rainure gravée. Les lignes visibles permettent ainsi de vérifier visuellement, lors du placement de l'objet-test, l'orthogonalité, la position dans l'espace (horizontalité et verticalité) et la coïncidence des nappes laser, celles-ci devant s'aligner sur les lignes visibles de l'objet.

Selon une caractéristique particulière de l'invention, les moyens d'alignement-positionnement de la sphère peuvent comporter en outre, de part et d'autre de chaque ligne équatoriale, des lignes visibles supplémentaires, avantageusement discontinues, parallèles aux trois lignes équatoriales, lesdites lignes visibles supplémentaires étant matérialisées par un trait peint ou imprimé, ou par une rainure gravée et lesdites lignes visibles supplémentaires étant formées à un espacement prédéterminé de la ligne équatoriale associée, de telle sorte qu'un alignement des moyens de repérage sur lesdites lignes visibles supplémentaires fournit une indication visuelle de la position de la bille en matériau de densité électronique d1 par rapport au point de concours des trois axes (V', H', C').

Ainsi, l'espacement entre les lignes visibles supplémentaires étant connu, l'opérateur peut en déduire, lorsque celles-ci sont alignées avec les moyens de repérage, la position de la bille en matériau de densité électronique d1, et donc la correction à apporter au placement de l'objet-test, sans autre appareil de mesure.

Conformément à une caractéristique particulière de l'invention, lorsque les moyens de repérage sont des nappes laser émises par des projecteurs laser, les lignes visibles et le cas échéant les lignes visibles supplémentaires peuvent être recouvertes d'une substance apte à réfléchir la lumière dans le spectre visible, pour s'éclairer de la même couleur que la couleur des nappes laser, lorsque lesdites lignes visibles, et lorsqu'elles sont présentes lesdites lignes visibles supplémentaires, sont alignées avec les nappes laser.

Les lignes visibles peuvent par exemple avantageusement être de couleur claire ou blanches. Ainsi, on facilite pour l'opérateur la confirmation visuelle de l'alignement des lignes visibles et des moyens de repérage, par réflexion de lumière.

Les lignes visibles et, le cas échéant, les lignes visibles supplémentaires, peuvent être pratiquées dans des zones équatoriales en forme de bande de la surface de la sphère, qui ont été traitées pour absorber ou diffuser la lumière dans le spectre visible. On accentue alors encore la confirmation visuelle de l'alignement des lignes visibles, éventuellement des lignes visibles supplémentaires avec les moyens de repérage, puisque la lumière des moyens de repérage est réfléchie sur les lignes visibles, et absorbée ou diffusée en dehors de ces lignes visibles.

Conformément à une caractéristique particulière de l'invention, les zones en forme de bande peuvent être formées par dépolissage ou peinture de couleur sombre.

La couleur sombre peut par exemple être du noir.

Ainsi, lorsque les moyens de repérage sont des nappes laser et que les lignes visibles sont de couleur claire, les lignes visibles reflètent la lumière laser lorsqu'elles sont alignées avec les nappes laser, tandis que dans les zones en forme de bande dépolies ou de couleur sombre la lumière laser est diffusée ou absorbée.

Conformément à une autre caractéristique particulière de l'invention, le diamètre de la sphère peut être choisi suffisamment grand pour que les moyens d'alignement-positionnement visuel puissent être distingués sans difficulté par l'oeil humain, le diamètre de ladite sphère étant notamment compris entre 80 et 200 mm, les lignes visibles et le cas échéant les lignes visibles supplémentaires ayant une largeur comprise entre 0,1 mm et 0,5 mm, notamment de 0,2 mm (largeur de ligne peinte ou de rainure), et les lignes visibles supplémentaires, lorsqu'elles sont présentes, étant espacées de 1 mm de part et d'autre de chaque ligne visible équatoriale, et les zones en forme de bande, lorsqu'elles sont présentes, ayant une largeur comprise entre 2 mm et 10 mm, notamment de 5mm, la bille ayant notamment un diamètre compris entre 2 et 10 mm, de préférence entre 5 et 6 mm.

Ainsi quand les nappes lasers sont en dehors des lignes visibles, les nappes lasers diffusent dans la zone en forme de bande dépolie ou sont absorbées dans la zone en forme de bande de couleur sombre. Par exemple, avec une zone en forme de bande de 5 mm de largeur, et des lignes visibles équatoriales et lignes visibles supplémentaires de largeur 0,2 mm, on peut, avec un espacement de 1 mm entre chaque ligne visible, placer dans chaque zone en forme de bande une ligne visible équatoriale, et deux lignes visibles supplémentaires de part et d'autre de chaque ligne visible équatoriale. Ainsi, dès que les nappes lasers sont sur une des cinq lignes visibles, les nappes lasers ne diffusent plus ou ne sont plus absorbées dans les zones en forme de bande et cette ligne visible s'éclaire. La couleur blanche est choisie de préférence pour les lignes visibles pour rendre ce système aussi performant avec des nappes laser vertes et qu'avec des nappes laser rouges. De plus, le fait de dépolir ou d'assombrir une zone en forme de bande de 5 mm évite que les nappes lasers d'un côté entrent dans la sphère transparente, subissent une diffraction et perturbent en sortie l'alignement d'une nappe laser de l'autre côté de la sphère (nappe laser controlatérale).

Ainsi, compte-tenu de la forme sphérique de l'objet-test et de sa symétrie par rotation, on assure que la forme de la sphère en matériau de densité électronique d2 (matière plastique) n'aura aucune influence sur l'image enregistrée de la bille en matériau de densité électronique d1, lors d'une quelconque rotation de l'appareil autour de l'un quelconque des trois axes de l'appareil de traitement, puisque l'épaisseur traversée de l'objet-test, en forme de sphère, est toujours la même. Cette géométrie permet donc d'obtenir des radiographies récurrentes homogènes, ce qui facilite les méthodes logicielles de détection automatique du centre de la bille et des limites du faisceau d'irradiation et améliore leur précision.

Avantageusement, la bille en matériau de densité électronique d1 peut être en tungstène.

Selon une caractéristique avantageuse de l'objet-test de la présente invention, les moyens de mise en place peuvent être constitués par un élément allongé métallique, de type tige ou tube, par exemple en titane ou en aluminium, dont une extrémité est destinée à être introduite dans un trou pratiqué dans la sphère et à y être solidarisée par exemple par vissage, et par une plaque qui est fixée sur l'autre extrémité de l'élément allongé et qui est agencée pour servir de contre-poids à l'objet-test et à l'élément allongé lorsque la plaque est posée sur le plan supérieur du support-patient et pour pouvoir, de là, être orientée pour le placement de l'objet-test au point de concours des trois axes (V', H', C').

La plaque peut comporter des moyens de réglage du niveau de ladite plaque par rapport au plan du support patient lors du placement de l'objet-test. Ces moyens peuvent notamment être des vis de mise à niveau.

Selon des caractéristiques particulières de l'invention, la plaque a une épaisseur comprise entre 20 et 40 mm, de préférence 20 mm, une largeur comprise entre 50 et 200 mm, de préférence 80 mm, et une longueur comprise entre 200 et 500 mm, de préférence 300 mm, l'élément allongé a une longueur comprise entre 50 et 300 mm, de préférence 100 mm, l'élément allongé pouvant être introduit dans la plaque sur une longueur comprise entre 20et 80 mm, de préférence 50 mm.

Lorsque l'élément allongé est une tige, son diamètre est compris entre 5 et 15 mm, et est de préférence de 10 mm maximum.

La sphère peut comporter un canal radial de diamètre légèrement supérieur à celui de la bille, ledit canal étant obturé par une tige en matériau de densité électronique d2 pouvant faire saillie de la surface de la sphère, et l'élément allongé peut être apte à se raccorder à la sphère par vissage à la périphérie de la tige, celui-ci pouvant comporter un évidement axial de forme complémentaire à la forme de la tige lorsque celle-ci est saillante, pour recevoir cette partie saillante.

La tige faisant saillie sert dans ce cas à renforcer le maintien de la sphère sur l'élément allongé. Elle a un diamètre compris entre 2 et 10 mm, de préférence de 8 mm, et une longueur comprise entre 60 et 120 mm, de préférence 70 mm.

Selon une caractéristique préférée de l'objet-test, l'élément allongé métallique peut être solidarisé à la sphère au niveau de l'intersection de deux lignes visibles équatoriales.

L'invention a également pour objet un procédé de fabrication d'un objet-test tel que défini ci-dessus, caractérisé par le fait qu'il comprend les étapes consistant à _{:}
- prendre une sphère en matériau de densité électronique d2et creuser avec une fraise à bout demi-sphérique, de diamètre égal à celui de la bille sphérique en matériau de densité électronique d1, un canal cylindrique depuis un point à la surface de la sphère, suivant un rayon de celle-ci, en creusant la sphère au-delà du centre de celle-ci sur une distance égale à la moitié du diamètre de la bille de matériau de densité électronique d1;
- introduire dans ce canal jusqu'au fond ladite bille;
- boucher le trou restant avec une tige en de densité électronique d2 de diamètre égal à celui de ladite bille, à l'extrémité de laquelle a été creusée la forme inverse de la demi bille, la tige étant alors collée dans le canal avec une matière adhésive de densité électronique d2, et la tige pouvant faire saillie de la surface de la sphère ;
- former une partie de plus grand diamètre à taraudage interne au voisinage de la surface de la sphère, à la base de la partie en saillie de la tige lorsque celle-ci fait saillie ;
- venir visser un élément allongé portant à une extrémité un filetage mâle sur le taraudage de la sphère, l'élément allongé comportant facultativement un évidement axial, pour le logement de la partie saillante de la tige lorsque celle-ci fait saillie ;
- fixer l'autre extrémité de l'élément allongé à une plaque ;
- former facultativement les zones équatoriales en forme de bande soit par dépolissage avec une machine outil 3D, soit par une peinture de couleur sombre, apte à diffuser ou absorber des longueurs d'onde dans le spectre visible, avantageusement de telle sorte que l'élément allongé est solidarisé à la sphère au centre de la région de recouvrement entre deux zones équatoriales en forme de bande ;
- former les lignes visibles et le cas échéant les lignes visibles supplémentaires par gravure ou impression sur la surface de la sphère avec une machine outil 3D, avantageusement de telle sorte que l'élément allongé est solidarisé à la sphère au point de concours de deux lignes visibles équatoriales ; et
- facultativement colorer les lignes visibles et le cas échéant les lignes visibles supplémentaires par peinture de celles-ci à la main au pinceau.

L'élément allongé peut être une tige pleine, auquel cas la tige en matériau de densité électronique d2 ne fait pas saillie de la sphère, l'élément allongé étant alors uniquement solidarisé à la sphère par vissage.

L'élément allongé peut également être un tube creux, pour alléger le poids de l'ensemble objet-test - tige.

Le fait que l'élément allongé comporte un évidement axial coopérant par emboîtement avec la partie saillante de la tige de matériau de densité électronique d2 renforce le maintien de la sphère sur l'élément allongé et peut avantageusement servir de guide pour l'opération de formation de la partie de plus grand diamètre à taraudage interne au voisinage de la surface de la sphère

L'invention a également pour objet un procédé de vérification de la coïncidence, de l'orthogonalité et de la position dans l'espace des moyens de repérage des trois axes de rotation (V', H', C') théoriques d'un appareil de traitement par radiothérapie dans une salle adaptée, la salle et/ou l'appareil de radiothérapie comportant des moyens de repérage de ces trois axes (V', H', C') théoriques, caractérisé par le fait qu'il comprend les opérations consistant à _{:}
- activer les moyens de repérage des trois axes (V', H', C') théoriques pour représenter visuellement les trois axes (V', H', C') théoriques ;
- placer un objet-test tel que défini ci-dessus au point de concours ou isocentre observé des trois axes théoriques (V', H', C') ;
- observer visuellement si les moyens de repérage des trois axes théoriques (V', H', C') suivent les moyens de d'alignement-positionnement respectifs correspondants sur l'objet-test ;
- en fonction de la ou des différences observées, modifier le réglage des moyens de repérage pour faire en sorte que les moyens de repérage des axes théoriques (V', H', C') suivent les moyens d'alignement-positionnement respectifs correspondants sur l'objet-test, afin d'assurer la coïncidence, l'orthogonalité et la position dans l'espace des moyens de repérage des trois axes théoriques.

Lorsque l'objet-test est placé sur le support-patient ayant un angle de rotation nul, la nappe sagittale est verticale et dans la direction longitudinale du support-patient, les deux nappes transversales sont dans le même plan, orthogonal au plan de la nappe sagittale et vertical, et les nappes frontales sont dans le même plan

L'invention a également pour objet un procédé de recherche de l'isocentre d'un appareil de traitement par radiothérapie, utilisant un objet-test tel que défini ci-dessus, des moyens externes et/ou portés par l'appareil étant prévus pour représenter visuellement les trois axes de rotation (V', H', C') théoriques de l'appareil, le procédé étant caractérisé par le fait qu'il comprend les opérations consistant à _{:}
- vérifier la coïncidence, l'orthogonalité et la position dans l'espace des moyens de repérage des trois axes théoriques (V', H', C') conformément au procédé défini ci-dessus ;
- l'objet-test restant à l'isocentre théorique (G'), irradier l'objet-test avec un faisceau de rayonnement émis à partir du collimateur, ledit faisceau étant détecté par les moyens de détection de rayonnement de l'appareil de traitement, et l'irradiation étant effectuée à différentes positions du support patient, du statif et du collimateur autour de chacun de leurs axes de rotation (V, H, C) ;
- analyser les images obtenues ;
- déterminer la position réelle des trois axes de rotation (V, H, C) et leur point de concours (G), isocentre réel de l'appareil de traitement par radiothérapie ;
- régler les moyens de repérage des trois axes théoriques (V', H', C') afin que les isocentres réel (G) et théorique (G') correspondent.

La présente invention a également pour objet un appareil de radiothérapie équipé de l'objet-test tel que défini ci-dessus.

Pour mieux illustrer l'objet de la présente invention, on va en décrire plus en détail ci-après deux modes de réalisation particuliers, avec référence au dessin annexé.

Sur ce dessin .
- la Figure 1 représente une vue de dessus de l'objet-test selon la présente invention, fixé à son support ;
- la Figure 2 représente une vue schématique de la surface de l'objet-test de la Figure 1 ;
- la Figure 3 représente une vue schématique d'un appareil de traitement par radiothérapie avec lequel l'objet-test de la présente invention est utilisé pour réaliser un test de W&L, les trois axes de rotation réels de l'appareil étant représentés ;
- la Figure 4 représente une vue analogue à la Figure 3, dans laquelle l'objet-test est en position d'utilisation, et dans laquelle des moyens de repérage matérialisent visuellement les trois axes de rotation théoriques de l'appareil de traitement par radiothérapie ; et
- la Figure 5 est une représentation schématique de l'objet-test.

La Figure 3 représente un appareil de traitement par radiothérapie classique, dans une salle de traitement par radiothérapie.

Cet appareil comporte, de manière classique, une structure qui comporte au moins une paroi verticale P portant un statif 1, et une table ou support patient 2.

Sur cette paroi verticale P est monté de manière classique mobile en rotation autour d'un axe horizontal le statif 1, sensiblement en forme de C vu de profil. Le statif 1 porte à une extrémité une tête d'irradiation qui se termine par un collimateur 3, et à l'autre extrémité, en face du collimateur 3 et tourné vers celui-ci, un dispositif de détection de rayonnement 4 émis à partir du collimateur 3, ce dispositif de détection de rayonnement étant appelé imageur portal 4, lequel permet de faire des clichés du rayonnement émis à partir du collimateur 3, et de réaliser le traitement informatique des clichés.

Le statif 1 est mobile en rotation à 360° autour d'un axe horizontal H, comme représenté sur la Figure 3, l'axe horizontal H passant sensiblement par le milieu de la partie dans le plan vertical du statif 1. L'angle de rotation nul du statif 1 correspond à la position verticale du statif 1, comme représenté sur la Figure 4.

Le collimateur 3, de manière classique pour les appareils de traitement par radiothérapie, possède un axe de rotation C autour de lui-même, ledit axe de rotation passant par le centre du collimateur 3, et ayant la direction du rayonnement émis à partir du collimateur 3.

La table ou support patient 2 possède également un axe de rotation vertical V, passant par le centre du collimateur 3 lorsque le statif 1 a un angle de rotation nul, et permet de déplacer le patient par rapport au statif pour irradier le patient sur différentes régions. L'angle de rotation nul du support patient 2 correspond à la position du support patient 2 dans laquelle l'axe de rotation horizontal H du statif 1 est parallèle à la direction longitudinale du support patient 2.

Ainsi, lorsque le statif 1 a un angle de rotation nul, comme par exemple cela est représenté sur la Figure 4, idéalement, l'axe de rotation C du collimateur 3 et l'axe de rotation V du support patient 2 sont confondus.

L'intersection des trois axes de rotation H, V, C constitue l'isocentre G réel de l'appareil de traitement par radiothérapie.

Si l'on se réfère maintenant à la Figure 4, on peut voir que l'on a représenté l'appareil de traitement par radiothérapie de la Figure 3, dans une position dans laquelle l'angle de rotation du statif 1 est nul, et l'angle de rotation du support patient 2 est nul.

Comme cela est représenté sur la Figure 4, des projecteurs laser 5, 6 sont prévus, d'une part sur une paroi P', orthogonale à la paroi P portant le statif 1, et d'autre part sur la paroi opposée à la paroi P, pour projeter des nappes laser, représentées hachurées sur la Figure 4. Il est à noter que sur la paroi opposée à la paroi P', non représentée pour faciliter la lecture de la Figure 4, est porté un projecteur laser identique au projecteur laser 5 porté par la paroi P', les deux projecteurs laser étant situés face à face et projetant dans les mêmes plans des nappes laser en direction de l'objet-test, ces plans étant définis ci-après en relation avec le projecteur laser 5. Il y a donc cinq nappes laser projetées en tout, trois étant représentées sur le dessin pour en faciliter la lecture.

Ces nappes laser servent à positionner l'isocentre théorique G' de l'appareil de traitement par radiothérapie.

Le projecteur laser 5 placé dans la paroi P', sensiblement au niveau de la surface supérieure du support patient 2, projette deux nappes laser orthogonales.

La première nappe laser a un plan horizontal et est appelée nappe frontale, et la deuxième nappe laser, appelée nappe transversale, a un plan vertical perpendiculaire au plan de la nappe frontale, et au plan de la paroi comprenant le projecteur laser 5.

Le projecteur laser 6, représenté schématiquement sur la Figure 4, est placé sur la paroi (non représentée pour faciliter la lecture du dessin) opposée à la paroi P, et projette une nappe laser verticale, appelée nappe sagittale, dont le plan est perpendiculaire aux plans respectifs des première et deuxième nappe, et qui constitue le plan médian dans la direction longitudinale du support patient 2, lorsque le support patient 2 a un angle de rotation nul, comme représenté sur la Figure 4.

L'intersection des nappes sagittale et frontale définit l'axe de rotation théorique H' du statif 1, tandis que l'intersection des nappes sagittale et transversale définit les axes de rotation théoriques du collimateur 3 C' et du support patient 2 V', lorsque le statif 1 a un angle de rotation nul.

L'intersection des trois nappes laser projetées par les projecteurs laser 5 et 6 (et par le projecteur laser porté par la paroi opposée à la paroi P', non représentée) définit donc l'isocentre théorique G' de l'appareil de traitement par radiothérapie.

Cet isocentre théorique G' sert à représenter, pour l'opérateur, la position du centre de la tumeur à traiter sur le patient. Pour un traitement optimal de la tumeur, c'est-à-dire un maximum de rayons sur la tumeur et un minimum sur les tissus avoisinant la tumeur, les deux isocentres G et G' doivent correspondre, comme indiqué plus haut.

L'isocentre réel G, fixé par les caractéristiques mécaniques de l'appareil de traitement, ne peut pas être déplacé par l'opérateur.

L'isocentre théorique G' est donc réglé par réglage des nappes laser pour correspondre à l'isocentre réel G.

Comme indiqué plus haut, la superposition de ces deux isocentres G et G' est réalisée au moyen du test de Winston & Lutz, qui consiste à placer une bille en matériau de densité électronique d1 élevée, dans le mode de réalisation présenté, en tungstène, à l'isocentre théorique G', à faire des images de cette bille par l'appareil de traitement par radiothérapie, et à analyser les images de la bille obtenues pour déterminer le décalage entre les deux isocentres G et G'.

La position de l'isocentre théorique G' est corrigée par réglage des nappes laser, jusqu'à ce que la position de l'isocentre théorique G' corresponde à la position de l'isocentre réel G.

Comme souligné plus haut, il est également important, pendant ce réglage, que les cinq nappes laser soient orthogonales deux à deux.

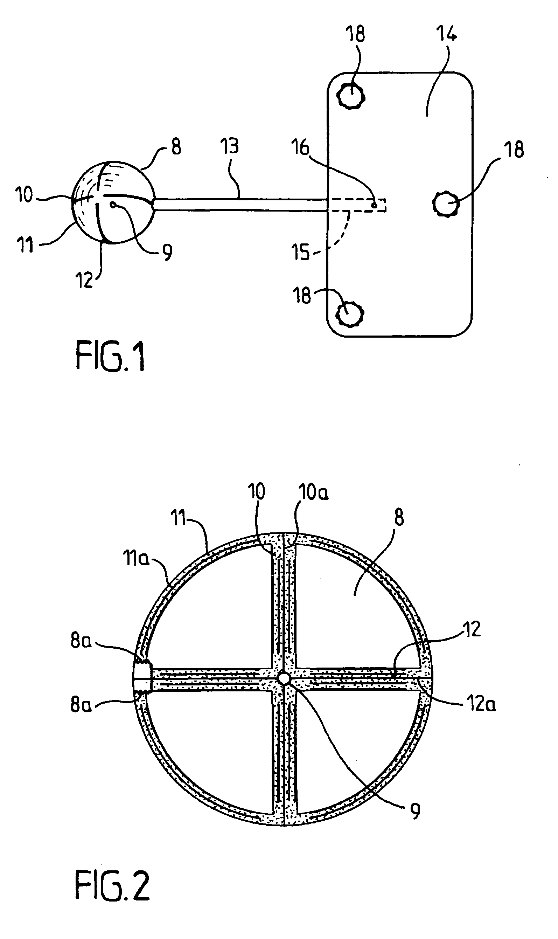

L'objet-test de la présente invention utilisé pour réaliser ce test est représenté sur les Figures 1, 2 et 5.

Il consiste en une sphère pleine 8 en matière de densité électronique d2 (d2 correspondant à une densité électronique relative de 1,156), telle que du poly(méthacrylate de méthyle) (PMMA), ayant un diamètre de 80 à 200 mm.

Au centre de cette sphère 8 est noyée une bille sphérique 9 en matériau de densité électronique d1, tel que du tungstène, la bille 9 ayant un diamètre de 5 mm, la densité électronique d1 du tungstène correspondant à une densité électronique relative de 13,995.

Une tige 8b de la même matière de densité électronique d2 que la sphère 8, fait saillie de la sphère 8 à partir du centre de celle-ci, la tige 8b ayant la direction d'un rayon de la sphère 8.

Trois zones en forme de bande 10, 11, 12, appelées bandes de fond, sont formées à la surface de la sphère 8.

Les trois bandes de fond 10, 11, 12 sont des bandes équatoriales orthogonales deux à deux, chaque bande de fond 10, 11, 12 étant formée par dépolissage de la surface de la sphère ou peinture de la surface de la sphère avec une couleur sombre sur une bande périphérique équatoriale de largeur 5 mm.

Les bandes de fond 10, 11, 12 absorbent ou diffusent la lumière dans le spectre visible.

La sphère 8 comporte trois jeux de lignes visibles équatoriales 10a, 11a, 12a, les lignes visibles étant matérialisées dans ce mode de réalisation par des rainures, les jeux étant orthogonaux deux à deux, et gravés à la surface de la sphère 8, au centre de chaque bande de fond respective 10, 11, 12, chaque jeu de rainures 10a, 11a, 12a ayant la même direction que la bande de fond respective 10, 11, 12 dans laquelle il est formé.

Chaque jeu de rainures 10a, 11a, 12a comporte une rainure équatoriale continue, parcourant l'entière circonférence de la sphère 8, ainsi que plusieurs rainures auxiliaires, parallèles à la rainure équatoriale, et situées de manière symétrique de part et d'autre de la rainure équatoriale, avec un espacement de 1 mm entre chaque rainure.

Les rainures de chaque jeu de rainures 10a, 11a, 12a, sont recouvertes d'une matière de couleur blanche, par exemple une peinture, reflétant la lumière dans le spectre visible, et de largeur de 0,2 mm. Chaque jeu de rainures 10a, 11a, 12a comporte une rainure équatoriale centrale et deux rainures auxiliaires de part et d'autre de la rainure équatoriale, les rainures dans chaque bande de fond étant espacées de 1 mm.

Les bandes de fond 10, 11, 12 et les jeux de rainures 10a, 11a, 12a sont agencés de telle sorte que la partie saillante de la tige 8b se trouve à l'intersection de deux rainures équatoriales, et donc à l'intersection de deux bandes de fond 10, 11, 12.

Comme cela est représenté sur les Figures 1 et 5, un élément allongé de type tige 13 en métal, tel que du titane ou de l'aluminium, porte à une extrémité un filetage mâle 13a, ladite tige 13 comportant au niveau de cette extrémité un évidement axial de forme complémentaire à la partie saillante de la tige 8b. La tige 13 est emboîtée dans la partie saillante de la tige 8b et vissée par vissage à un taraudage 8a porté par la sphère 8 au voisinage de sa surface, à la base de la partie saillante de la tige 8b.

Un support 14, constitué par une plaque rectangulaire en plastique dense (type polyoxyméthylène vendu sous la marque commerciale DELRIN^{®}) à bords arrondis, de 20 mm d'épaisseur, porte sur un de ses côtés d'épaisseur un alésage 15, à l'intérieur duquel est fixée l'autre extrémité de la tige 13.

Un trou traversant 16 est pratiqué à travers la dimension d'épaisseur du support 14, au niveau de l'extrémité interne de l'alésage 15, le trou 16 permettant le passage transversal d'un axe métallique, afin de bloquer en position la tige 13 et d'éviter tout mouvement de rotation par rapport au support 14 de la tige 13, et donc de la sphère 8.

Le support 14 comporte en outre des vis de mise à niveau 18, permettant de régler la hauteur et l'inclinaison du support 14 par rapport à la surface supérieure du support-patient 2 sur laquelle il est posé dans le cadre du test de Winston-Lutz.

Lorsque le support 14 est placé de manière adéquate sur le support-patient, notamment centré et de niveau, une première rainure équatoriale est dans le plan moyen du support, donc horizontale, une deuxième rainure équatoriale est verticale, dans l'axe de la tige 13, la troisième rainure équatoriale étant orthogonale aux deux autres, verticale.

En utilisation pour le test de Winston-Lutz, l'objet-test constitué par la sphère 8, est fixé par vissage des filetages 8a de la sphère 8 et 13a de la tige 13, la tige 13 étant elle-même fixée au support 14, de telle sorte qu'au moins une des rainures équatoriales d'un des jeux de rainures 10a, 11a, 12a appartient au plan moyen du support 14, comme cela est représenté sur la Figure 1.

Comme indiqué sur la Figure 4, le support 14 est posé à l'extrémité du support patient 2 tournée vers le statif 1, de telle sorte que seul le support 14 repose sur la surface supérieure du support patient 2, la tige 13 et la sphère 8 étant en porte-à-faux au-dessus de l'imageur portal 4.

Les projecteurs laser 5 et 6 (ainsi que le projecteur laser non représenté en face du projecteur laser 5) sont ensuite activés, de façon à projeter les nappes laser sagittale, frontales et transversales.

La position de la sphère 8 est réglée de telle sorte que chaque rainure équatoriale de chaque jeu de rainure 10a, 11a, 12a est alignée sur une des cinq nappes laser. Si les nappes laser ne sont pas rigoureusement orthogonales deux à deux, ce qui se constate si on ne parvient pas à placer la sphère 8 simultanément sur les cinq nappes de telle sorte que chaque rainure équatoriale de chaque jeu de rainure 10a, 11a, 12a corresponde à une nappe laser respective, il faut alors ajuster les projecteurs 5, 6 pour faire en sorte de respecter cette orthogonalité des cinq nappes laser.

Si les nappes lasers ne sont pas alignées sur une des rainures blanches de chaque jeu de rainure 10a, 11a, 12a gravées sur la surface de la sphère 8 dans les bandes de fond 10, 11, 12, les lasers diffusent ou sont absorbés dans la bande de fond respective 10, 11, 12. Si en revanche les nappes lasers sont alignées sur une des rainures blanches, alors les lasers ne diffusent plus ou ne sont plus absorbés par la bande de fond 10, 11, 12, mais sont réfléchis par la rainure blanche, et la rainure blanche prend la couleur de la nappe laser, rouge ou verte dans le cas de la plupart des lasers utilisés.

Une fois que la sphère 8 est correctement placée à l'intersection des cinq nappes laser, le test de Winston Lutz proprement dit, comme indiqué plus haut, peut débuter, la sphère 8 étant placée à l'isocentre théorique G', comme cela est représenté sur la Figure 4.

Il est à noter que d'autres moyens de support de la sphère 8 pourraient être envisagés, sans s'écarter de la portée de la présente invention.

Le procédé de fabrication de l'objet-test est le suivant : on prend une sphère 8 en PMMA de diamètre 100 mm et on creuse avec une fraise de diamètre 5 mm à bout demi-sphérique un canal cylindrique depuis un point (pôle) à la surface de la sphère 8, suivant un rayon de celle-ci, en creusant la sphère 8 au-delà du centre de celle-ci de 2,5 mm.

On introduit dans ce canal jusqu'au fond une bille 9 en tungstène de 5 mm de diamètre.

On bouche ensuite le trou restant avec une tige 8b en PMMA de 5 mm de diamètre à l'extrémité de laquelle a été creusée la forme inverse de celle de la demi bille 9 en tungstène, ce qui permet à la tige 8b de combler parfaitement le canal creusé dans la sphère 8. La tige 8b est alors collée dans le canal avec une colle spéciale pour PMMA.

On forme alors une partie de plus grand diamètre à taraudage interne au voisinage de la surface de la sphère 8, à la base de la partie en saillie de la tige 8b, la tige 8b faisant saillie de la sphère 8 de 40 mm environ.

On vient ensuite visser une tige 13 portant à une extrémité un filetage mâle 13a sur le taraudage 8a de la sphère 8, la tige 13 étant une tige pleine comportant un évidement axial pour le logement de la partie saillante de la tige (8b).

L'emboîtement de la tige 8b dans l'évidement de la tige 13 renforce le maintien de la sphère sur la tige 13, et peut servir de guide pour l'opération consistant à former le taraudage 8a sur la sphère.

On fixe ensuite l'autre extrémité de la tige (13) à une plaque (14), puis on forme facultativement les zones en forme de bande (10, 11, 12) soit par dépolissage avec une machine outil 3D, soit par une peinture de couleur sombre, par exemple de couleur noire, apte à diffuser ou absorber des longueurs d'onde dans le spectre visible, avantageusement de telle sorte que la tige (13) soit placée au centre de la région de recouvrement entre deux zones en forme de bande (10, 11, 12).

On forme alors les lignes visibles et le cas échéant les lignes visibles supplémentaires (10a, 11a, 12a) par gravure ou impression sur la surface de la sphère (8) avec une machine outil 3D, avantageusement de telle sorte que la tige (13) est placée au point de concours de deux lignes visibles équatoriales, et facultativement, on colore les lignes visibles et le cas échéant les lignes visibles supplémentaires (10a, 11a, 12a) par peinture de celles-ci à la main au pinceau, par exemple avec une peinture de couleur blanche ou claire, réfléchissant les longueurs d'onde dans le spectre visible.

Ces dernières opérations peuvent être éventuellement réalisées en mettant la sphère 8 en rotation autour du tube 13 de support en aluminium.

## Revendications

1. Objet-test pour un test de contrôle qualité d'un appareil de traitement par radiothérapie, comprenant une bille sphérique (9) faite d'un matériau ayant une densité électronique d1, la bille sphérique (9) de densité électronique d1 étant disposée au centre d'une sphère (8) en un matériau ayant une densité électronique d2 afin de constituer avec elle l'objet-test, le rapport de la densité électronique d1 sur la densité électronique d2 étant supérieur ou égal à 1,1, ladite sphère (8) comportant des moyens (8a) permettant de la rendre solidaire de moyens (13, 14) de mise en place de l'objet-test sur l'appareil de traitement par radiothérapie, ladite bille ayant un diamètre compris entre 2 et 10 mm, de préférence entre 5 et 6 mm, **caractérisé par le fait que** le diamètre de ladite sphère (8) est compris entre 80 et 200 mm, et ladite sphère (8) porte sur sa surface externe des moyens (10a, 11a, 12a) d'alignement visuel constitués par trois lignes équatoriales visibles, orthogonales deux à deux, formées sur la surface de la sphère (8) et permettant un positionnement de l'objet-test.

2. Objet-test selon la revendication 1, **caractérisé par le fait que** le matériau de densité électronique d2 à partir duquel est fabriquée la sphère (8) est une matière plastique, en particulier une matière plastique transparente pour la lumière visible, notamment en poly(méthacrylate de méthyle) (PMMA).

3. Objet-test selon l'une des revendications 1 ou 2, **caractérisé par le fait que** les moyens d'alignement-positionnement (10a, 11a, 12a) formés sur la surface de la sphère (8) en matériau de densité électronique d2 sont matérialisés par un trait peint ou imprimé, ou par une rainure gravée.

4. Objet-test selon la revendication 3, **caractérisé par le fait que** les moyens d'alignement-positionnement (10a, 11a, 12a) de la sphère (8) comportent en outre, de part et d'autre de chaque ligne équatoriale, des lignes visibles supplémentaires, avantageusement discontinues, parallèles aux trois lignes équatoriales, lesdites lignes visibles supplémentaires étant matérialisées par un trait peint ou imprimé, ou par une rainure gravée et lesdites lignes visibles supplémentaires étant formées à un espacement prédéterminé de la ligne équatoriale associée.

5. Objet-test selon l'une des revendications 3 ou 4, **caractérisé par le fait que** les lignes visibles et le cas échéant les lignes visibles supplémentaires sont recouvertes d'une substance apte à réfléchir la lumière dans le spectre visible.

6. Objet-test selon l'une des revendications 3 à 5, **caractérisé par le fait que** les lignes visibles et, le cas échéant, les lignes visibles supplémentaires, sont pratiquées dans des zones équatoriales en forme de bande de la surface de la sphère, qui ont été traitées pour absorber ou diffuser la lumière dans le spectre visible.

7. Objet-test selon la revendication 6, **caractérisé par le fait que** les zones en forme de bande (10, 11, 12) ont été formées par dépolissage ou peinture de couleur sombre.

8. Objet-test selon l'une des revendications 4 à 7, **caractérisé par le fait que** les lignes visibles et le cas échéant les lignes visibles supplémentaires ont une largeur comprise entre 0,1 mm et 0,5 mm, notamment de 0,2 mm, et les lignes visibles supplémentaires, lorsqu'elles sont présentes, sont espacées de 1 mm de part et d'autre de chaque ligne visible équatoriale, et les zones en forme de bande, lorsqu'elles sont présentes, ont une largeur comprise entre 2 mm et 10 mm, notamment de 5mm.

9. Objet-test selon l'une des revendications 1 à 8, **caractérisé par le fait que** le matériau de densité électronique d1 constituant la bille (9) est le tungstène.

10. Objet-test selon l'une des revendications 1 à 9, **caractérisé par le fait que** les moyens (13, 14) de mise en place sont constitués par un élément allongé métallique (13), de type tige ou tube, par exemple en titane ou en aluminium, dont une extrémité est destinée à être introduite dans un trou (8a) pratiqué dans la sphère (8) et à y être solidarisée par exemple par vissage, et par une plaque (14) qui est fixée sur l'autre extrémité de l'élément allongé (13) et qui est agencée pour servir de contre-poids à l'objet-test et à l'élément allongé (13) lorsque la plaque (14) est disposée par rapport à l'appareil de traitement par radiothérapie.

11. Objet-test selon la revendication 10, **caractérisé par le fait que** la plaque (14) comporte des moyens (18) de réglage du niveau de ladite plaque (14) par rapport au plan d'un support patient (2) de l'appareil de traitement par radiothérapie lors du placement de l'objet-test.

12. Objet-test selon l'une des revendications 10 ou 11, **caractérisé par le fait que** la sphère (8) comporte un canal radial de diamètre légèrement supérieur à celui de la bille (9), ledit canal étant obturé par une tige (8b) en matériau de densité électronique d2 pouvant faire saillie de la surface de la sphère (8), et **par le fait que** l'élément allongé (13) est apte à se raccorder à la sphère par vissage à la périphérie de la tige (8b), celui-ci pouvant comporter un évidement axial de forme complémentaire à la forme de la tige (8b) lorsque celle-ci est saillante, pour recevoir cette partie saillante.

13. Objet-test selon l'une des revendications 10 à 12, **caractérisé par le fait que** l'élément allongé (13) est solidarisé à la sphère (8) au niveau de l'intersection de deux lignes visibles équatoriales (10a, 11a, 12a).

14. Procédé de fabrication d'un objet-test tel que défini à l'une quelconque des revendications 1 à 13, **caractérisé par le fait qu'**il comprend les étapes consistant à :
- prendre une sphère (8) en matériau de densité électronique d2 et creuser avec une fraise à bout demi-sphérique, de diamètre égal à celui de la bille sphérique (9) en matériau de densité électronique d1, un canal cylindrique depuis un point à la surface de la sphère (8), suivant un rayon de celle-ci, en creusant la sphère (8) au-delà du centre de celle-ci sur une distance égale à la moitié du diamètre de la bille (9) de matériau de densité électronique d1;
- introduire dans ce canal jusqu'au fond ladite bille (9);
- boucher le trou restant avec une tige (8b) en matériau de densité électronique d2 de diamètre égal à celui de ladite bille (9), à l'extrémité de laquelle a été creusée la forme inverse de la demi bille (9), la tige (8b) étant alors collée dans le canal avec une matière adhésive de densité électronique d2, et la tige (8b) pouvant faire saillie de la surface de la sphère (8) ;
- former une partie de plus grand diamètre à taraudage interne au voisinage de la surface de la sphère (8), à la base de la partie en saillie de la tige (8b) lorsque celle-ci fait saillie ;
- venir visser un élément allongé (13) portant à une extrémité un filetage mâle (13a) sur le taraudage (8a) de la sphère (8), l'élément allongé (13) comportant facultativement un évidement axial, pour le logement de la partie saillante de la tige (8b) lorsque celle-ci fait saillie ;
- fixer l'autre extrémité de l'élément allongé (13) à une plaque (14) ;
- former facultativement les zones équatoriales en forme de bande (10, 11, 12) soit par dépolissage avec une machine outil 3D, soit par une peinture de couleur sombre, apte à diffuser ou absorber des longueurs d'onde dans le spectre visible, avantageusement de telle sorte que l'élément allongé (13) est solidarisé à la sphère au centre de la région de recouvrement entre deux zones «équatoriales en forme de bande (10, 11, 12) ;
- former les lignes visibles et le cas échéant les lignes visibles supplémentaires (10a, 11a, 12a) par gravure ou impression sur la surface de la sphère (8) avec une machine outil 3D, avantageusement de telle sorte que l'élément allongé (13) est solidarisé à la sphère au point de concours de deux lignes visibles équatoriales ; et
- facultativement colorer les lignes visibles et le cas échéant les lignes visibles supplémentaires (10a, 11a, 12a) par peinture de celles-ci à la main au pinceau.

15. Procédé de vérification de la coïncidence, de l'orthogonalité et de la position dans l'espace d'une salle de traitement des moyens de repérage (5, 6) de l'isocentre d'un appareil de traitement par radiothérapie, ledit isocentre étant le point d'intersection des trois axes de rotation (V', H', C') théoriques de l'appareil de traitement par radiothérapie, **caractérisé par le fait qu'**il comprend les opérations consistant à :
- activer les moyens de repérage (5, 6) des trois axes (V', H', C') théoriques pour représenter visuellement les trois axes (V', H', C') théoriques ;
- placer un objet-test tel que défini à l'une quelconque des revendications 1 à 13 au point de concours ou isocentre observé (G') des trois axes théoriques (V', H' , C') ;
- observer visuellement si chacun des moyens de repérage (5, 6) des trois axes théoriques (V', H', C') suivent les moyens d'alignement-positionnement (10a, 11a, 12a) respectifs correspondants sur l'objet-test ;
- en fonction de la ou des différences observées, modifier le réglage des moyens de repérage (5, 6) pour faire en sorte que les moyens de repérage (5,6) des axes théoriques (V', H', C') suivent les moyens d'alignement-positionnement (10a, 11a, 12a) respectifs correspondants sur l'objet-test, afin d'assurer la coïncidence, l'orthogonalité et la position dans l'espace des moyens de repérage (5, 6) des trois axes théoriques (V', H', C').

16. Procédé de recherche de l'isocentre d'un appareil de traitement par radiothérapie, utilisant un objet-test tel que défini à l'une quelconque des revendications 1 à 13, des moyens externes et/ou portés par l'appareil étant prévus pour représenter visuellement les trois axes de rotation (V', H', C') théoriques de l'appareil et leur point d'intersection ou isocentre, le procédé étant **caractérisé par le fait qu'**il comprend les opérations consistant à :
- vérifier la coïncidence, l'orthogonalité et la position dans l'espace des moyens de repérage (5, 6) des trois axes théoriques (V', H', C') conformément au procédé défini à la revendication 15 ;
- l'objet-test restant à l'isocentre théorique (G'), irradier l'objet-test avec un faisceau de rayonnement émis à partir du collimateur (3), ledit faisceau étant détecté par les moyens de détection de rayonnement (4) de l'appareil de traitement, et l'irradiation étant effectuée à différentes positions du support patient (2), du statif (1) et du collimateur (3) autour de chacun de leurs axes de rotation (V, H, C) ;
- analyser les images obtenues ;
- déterminer la position réelle des trois axes de rotation (V, H, C) et leur point de concours (G), isocentre réel de l'appareil de traitement par radiothérapie ;
- régler les moyens de repérage (5, 6) des trois axes théoriques (V', H', C') afin que les isocentres réel (G) et théorique (G') correspondent.

## Patentansprüche

1. Testobjekt für einen Test zur Qualitätskontrolle eines Geräts zur Strahlentherapie-Behandlung, das ein rundes Kügelchen (9) umfasst, das aus einem Material hergestellt ist, das eine Elektronendichte d1 aufweist, wobei das runde Kügelchen (9) mit der Elektronendichte d1 in der Mitte einer Kugel (8) aus einem Material mit einer Elektronendichte d2 angeordnet ist, um mit ihr das Testobjekt zu bilden, wobei das Verhältnis der Elektronendichte d1 zur Elektronendichte d2 größer oder gleich 1,1 beträgt, wobei die Kugel (8) Mittel (8a) aufweist, die ermöglichen, sie fest mit Mitteln (13, 14) zum Platzieren des Testobjekts an dem Gerät zur Strahlentherapie-Behandlung zu verbinden, wobei das Kügelchen einen Durchmesser zwischen 2 und 10 mm, vorzugsweise zwischen 5 und 6 mm aufweist, **dadurch gekennzeichnet, dass** der Durchmesser der Kugel (8) zwischen 80 und 200 mm liegt, und die Kugel (8) auf ihrer Außenfläche Mittel (10a, 11a, 12a) zum optischen Ausrichten trägt, die aus drei sichtbaren, paarweise orthogonalen Äquatoriallinien gebildet sind, die auf der Oberfläche der Kugel (8) ausgebildet sind und eine Positionierung des Testobjekts ermöglichen.

2. Testobjekt nach Anspruch 1, **dadurch gekennzeichnet, dass** das Material mit der Elektronendichte d2, aus dem die Kugel (8) hergestellt ist, ein Kunststoff ist, insbesondere ein Kunststoff, der für sichtbares Licht durchlässig ist, insbesondere aus Polymethylmethacrylat (PMMA).

3. Testobjekt nach einem der Ansprüche 1 oder 2, **dadurch gekennzeichnet, dass** die Ausrichtungs- und Positionierungsmittel (10a, 11a, 12a), die auf der Oberfläche der Kugel (8) aus dem Material mit der Elektronendichte d2 ausgebildet sind, durch einen aufgezeichneten oder aufgedruckten Strich oder durch eine eingeritzte Rille realisiert sind.

4. Testobjekt nach Anspruch 3, **dadurch gekennzeichnet, dass** die Ausrichtungs- und Positionierungsmittel (10a, 11a, 12a) der Kugel (8) ferner auf beiden Seiten jeder Äquatoriallinie zusätzliche sichtbare, vorteilhafterweise unterbrochene Linien parallel zu den drei Äquatoriallinien aufweisen, wobei die zusätzlichen sichtbaren Linien durch einen aufgezeichneten oder aufgedruckten Strich oder durch eine eingeritzte Rille realisiert sind und die zusätzlichen sichtbaren Linien in einem festgelegten Abstand zur zugehörigen Äquatoriallinie ausgebildet sind.

5. Testobjekt nach einem der Ansprüche 3 oder 4, **dadurch gekennzeichnet, dass** die sichtbaren Linien und gegebenenfalls die zusätzlichen sichtbaren Linien mit einer Substanz überzogen sind, die in der Lage ist, Licht im sichtbaren Spektrum zu reflektieren.

6. Testobjekt nach einem der Ansprüche 3 bis 5, **dadurch gekennzeichnet, dass** die sichtbaren Linien und gegebenenfalls die zusätzlichen sichtbaren Linien in streifenförmigen Äquatorialbereichen der Kugeloberfläche vorgesehen sind, die behandelt wurden, um Licht im sichtbaren Spektrum zu absorbieren oder zu streuen.

7. Testobjekt nach Anspruch 6, **dadurch gekennzeichnet, dass** die streifenförmigen Bereiche (10, 11, 12) durch Mattieren oder Aufbringen einer dunklen Farbe gebildet wurden.

8. Testobjekt nach einem der Ansprüche 4 bis 7, **dadurch gekennzeichnet, dass** die sichtbaren Linien und gegebenenfalls die zusätzlichen sichtbaren Linien eine Breite zwischen 0,1 mm und 0,5 mm, insbesondere von 0,2 mm aufweisen, und die zusätzlichen sichtbaren Linien, wenn sie vorhanden sind, auf beiden Seiten jeder sichtbaren Äquatoriallinie einen Abstand von 1 mm aufweisen, und die streifenförmigen Bereiche, wenn sie vorhanden sind, eine Breite zwischen 2 mm und 10 mm, insbesondere von 5 mm aufweisen.

9. Testobjekt nach einem der Ansprüche 1 bis 8, **dadurch gekennzeichnet, dass** das Material mit der Elektronendichte d1, das das Kügelchen (9) bildet, Wolfram ist.

10. Testobjekt nach einem der Ansprüche 1 bis 9, **dadurch gekennzeichnet, dass** die Platzierungsmittel (13, 14) aus einem länglichen Metallelement (13) in der Art eines Stabs oder einer Röhre, beispielsweise aus Titan oder aus Aluminium, gebildet sind, wovon ein Ende dafür bestimmt ist, in eine Bohrung (8a) gesteckt zu werden, die in die Kugel (8) eingebracht ist, und dort beispielsweise durch Verschrauben fest verbunden zu werden, und aus einer Platte (14), die an dem anderen Ende des länglichen Elements (13) befestigt ist und angeordnet ist, um als Gegengewicht zu dem Testobjekt und dem länglichen Element (13) zu dienen, wenn die Platte (14) bezogen auf das Gerät zur Strahlentherapie-Behandlung angeordnet ist.

11. Testobjekt nach Anspruch 10, **dadurch gekennzeichnet, dass** die Platte (14) Mittel (18) zum Einstellen der Höhe der Platte (14) bezogen auf die Ebene einer Patientenliege (2) des Geräts zur Strahlentherapie-Behandlung beim Platzieren des Testobjekts aufweist.

12. Testobjekt nach einem der Ansprüche 10 oder 11, **dadurch gekennzeichnet, dass** die Kugel (8) einen radialen Kanal mit einem Durchmesser etwas größer als der des Kügelchens (9) aufweist, wobei der Kanal mit einem Stab (8b) aus einem Material mit der Elektronendichte d2 verschlossen ist, der aus der Oberfläche der Kugel (8) herausragen kann, und dadurch, dass das längliche Element (13) in der Lage ist, mit der Kugel durch Aufschrauben am Umfang des Stabs (8b) verbunden zu werden, wobei dieses eine axiale Aussparung aufweisen kann, deren Form auf die Form des Stabs (8b) abgestimmt ist, wenn dieser herausragt, um diesen herausragenden Teil aufzunehmen.

13. Testobjekt nach einem der Ansprüche 10 bis 12, **dadurch gekennzeichnet, dass** das längliche Element (13) mit der Kugel (8) an dem Schnittpunkt von zwei sichtbaren Äquatoriallinien (10a, 11a, 12a) fest verbunden ist.

14. Verfahren zur Herstellung eines Testobjekts nach einem der Ansprüche 1 bis 13, **dadurch gekennzeichnet, dass** es folgende Schritte umfasst:
- Verwenden einer Kugel (8) aus einem Material mit der Elektronendichte d2 und Einbringen, mit einem Radiusfräser mit demselben Durchmesser wie dem des runden Kügelchens (9) aus einem Material mit der Elektronendichte d1, eines zylindrischen Kanals von einer Stelle an der Oberfläche der Kugel (8) aus, entlang einem Radius derselben, durch Aushöhlen der Kugel (8) über die Mitte derselben hinaus über einen Abstand, der der Hälfte des Durchmessers des Kügelchen (9) aus dem Material mit der Elektronendichte d1 entspricht;
- Einbringen des Kügelchens (9) bis ganz unten in diesen Kanal;
- Verschließen der verbleibenden Bohrung mit einem Stab (8b) aus einem Material mit der Elektronendichte d2 mit demselben Durchmesser wie dem des Kügelchens (9), an dessen Ende die umgekehrte Form des Halbkügelchens (9) eingebracht wurde, wobei der Stab (8b) dann mit einem Klebstoff mit der Elektronendichte d2 in den Kanal geklebt wird und der Stab (8b) aus der Oberfläche der Kugel (8) ragen kann;
- Bilden eines Teils mit einem größeren Durchmesser mit Innengewinde in der Nähe der Oberfläche der Kugel (8), am Fuß des herausragenden Teils des Stabs (8b), wenn dieser herausragt;
- Schrauben eines länglichen Elements (13), das an einem Ende ein Außengewinde (13a) aufweist, auf das Gewinde (8a) der Kugel (8), wobei das längliche Element (13) wahlweise eine axiale Aussparung für die Aufnahme des herausragenden Teils des Stabs (8b) aufweist, wenn dieser herausragt;
- Befestigen des anderen Endes des länglichen Elements (13) an einer Platte (14);
- wahlweise Bilden der streifenförmigen Äquatorialbereiche (10, 11, 12) entweder durch Mattieren mit einer 3D-Werkzeugmaschine oder durch Auftragen einer dunklen Farbe, die in der Lage ist, Wellenlängen im sichtbaren Spektrum zu streuen oder zu absorbieren, vorteilhafterweise derart, dass das längliche Element (13) mit der Kugel in der Mitte des Überdeckungsbereichs zwischen zwei streifenförmigen Äquatorialbereichen (10, 11, 12) fest verbunden wird;
- Bilden der sichtbaren Linien und gegebenenfalls der zusätzlichen sichtbaren Linien (10a, 11a, 12a) durch Einritzen in oder Aufdrucken auf die Oberfläche der Kugel (8) mit einer 3D-Werkzeugmaschine, vorteilhafterweise derart, dass das längliche Element (13) mit der Kugel am Punkt des Zusammentreffens von zwei sichtbaren Äquatoriallinien fest verbunden wird; und
- wahlweise Färben der sichtbaren Linien und gegebenenfalls der zusätzlichen sichtbaren Linien (10a, 11a, 12a) durch Bemalen derselben per Hand mit einem Pinsel.

15. Verfahren zum Überprüfen der Übereinstimmung, Orthogonalität und räumlichen Lage in einem Behandlungsraum von Markierungsmitteln (5, 6) des Isozentrums eines Geräts zur Strahlentherapie-Behandlung, wobei das Isozentrum der Schnittpunkt der drei theoretischen Drehachsen (V', H', C') des Geräts zur Strahlentherapie-Behandlung ist, **dadurch gekennzeichnet, dass** es folgende Vorgänge umfasst:
- Anschalten der Markierungsmittel (5, 6) der drei theoretischen Achsen (V', H', C'), um die drei theoretischen Achsen (V', H', C') optisch darzustellen;
- Platzieren eines Testobjekts nach einem der Ansprüche 1 bis 13 an dem beobachteten Punkt des Zusammentreffens oder Isozentrum (G') der drei theoretischen Achsen (V', H', C');
- optisch Feststellen, ob jedes der Markierungsmittel (5, 6) der drei theoretischen Achsen (V', H', C') den jeweiligen entsprechenden Ausrichtungs- und Positionierungsmitteln (10a, 11a, 12a) an dem Testobjekt folgt;
- in Abhängigkeit von dem oder den beobachteten Unterschied(en) Ändern der Einstellung der Markierungsmittel (5, 6), damit die Markierungsmittel (5, 6) der theoretischen Achsen (V', H', C') den jeweiligen entsprechenden Ausrichtungs- und Positionierungsmitteln (10a, 11a, 12a) an dem Testobjekt folgen, um die Übereinstimmung, Orthogonalität und räumliche Lage der Markierungsmittel (5, 6) der drei theoretischen Achsen (V', H', C') zu gewährleisten.

16. Verfahren zum Suchen nach dem Isozentrum eines Geräts zur Strahlentherapie-Behandlung unter Verwendung eines Testobjekts nach einem der Ansprüche 1 bis 13, wobei äußere Mittel und/oder Mittel, die das Gerät trägt, für die optische Darstellung der drei theoretischen Drehachsen (V', H', C') des Geräts und ihres Schnittpunkts oder Isozentrums vorgesehen sind, wobei das Verfahren **dadurch gekennzeichnet ist, dass** es folgende Vorgänge umfasst:
- Überprüfen der Übereinstimmung, Orthogonalität und räumlichen Lage von Markierungsmitteln (5, 6) der drei theoretischen Achsen (V', H', C') gemäß dem Verfahren nach Anspruch 15;
- während das Testobjekt im theoretischen Isozentrum (G') bleibt, Bestrahlen des Testobjekts mit einem Strahlenbündel, das von dem Kollimator (3) ausgesendet wird, wobei das Bündel von den Strahlennachweismitteln (4) des Behandlungsgeräts erfasst wird und die Bestrahlung in unterschiedlichen Positionen der Patientenliege (2), des Stativs (1) und des Kollimators (3) um jede ihrer Drehachsen (V, H, C) erfolgt;
- Analysieren der erhaltenen Bilder;
- Bestimmen der tatsächlichen Position der drei Drehachsen (V, H, C) und ihres Punkts des Zusammentreffens (G), dem tatsächlichen Isozentrum des Geräts zur Strahlentherapie-Behandlung;
- Einstellen der Markierungsmittel (5, 6) der drei theoretischen Achsen (V', H', C'), damit das tatsächliche (G) und theoretische (G') Isozentrum übereinstimmen.

## Claims

1. A test-object for a quality control test of a radiotherapy treatment apparatus, comprising a spherical ball (9) made of a material having an electron density d1, the spherical ball (9) having the electron density d1 being provided at the center of a sphere (8) made of a material having an electron density d2 in order to constitute therewith the test-object, the ratio of the electron density d1 to the electron density d2 being greater than or equal to 1.1, said sphere (8) comprising means (8a) for securing it to means (13, 14) for placing the test-object on the radiotherapy treatment apparatus, said ball having a diameter between 2 and 10 mm, preferably between 5 and 6 mm, **characterized by** the fact that the diameter of said sphere (8) is between 80 and 200 mm, and said sphere (8) is provided, on its outer surface, with visual alignment means (10a, 11a, 12a) constituted by three visible equatorial lines, orthogonal in pairs, formed on the surface of the sphere (8) and allowing a positioning of the test-object.

2. The test-object according to claim 1, **characterized by** the fact that the material having an electron density d2 from which the sphere (8) is made is a plastic material, particularly a plastic material transparent to visible light, especially made of poly(methyl methacrylate) (PMMA).

3. The test-object according to one of claims 1 or 2, **characterized by** the fact that the alignment-positioning means (10a, 11a, 12a) formed on the surface of the sphere (8) made of a material having an electron density d2 are materialized by a painted or printed line, or by an etched groove.

4. The test-object according to claim 3, **characterized by** the fact that the alignment-positioning means (10a, 11a, 12a) of the sphere (8) further comprise, on both sides of each equatorial line, additional visible lines, advantageously dashed lines, parallel to the three equatorial lines, said additional visible lines being materialized by a painted or printed line, or by an etched groove and said additional visible lines being formed at a predetermined spacing from the corresponding equatorial line.

5. The test-object according to any one of claims 3 or 4, **characterized by** the fact that the visible lines, and if applicable the additional visible lines, are coated with a substance able to reflect the light in the visible spectrum.

6. The test-object according to any one of claims 3 to 5, **characterized by** the fact that the visible lines, and if applicable the additional visible lines, are made in strip-shaped equatorial areas of the sphere surface, which are treated to absorb or scatter the light in the visible spectrum.

7. The test-object according to claim 6, **characterized by** the fact that the strip-shaped areas (10, 11, 12) are formed by dull rubbing or by painting in a dark color.

8. The test-object according to any one of claims 4 to 7, **characterized by** the fact that the visible lines, and if applicable the additional visible lines, have a width between 0.1 mm and 0.5 mm, especially of 0.2 mm, and the additional visible lines, when they are present, are spaced apart by 1 mm on both sides of each visible equatorial line, and the strip-shaped areas, when they are present, have a width between 2 mm and 10 mm, especially of 5 mm.

9. The test-object according to any one of claims 1 to 8, **characterized by** the fact that the material having an electron density d1 constituting the ball (9) is tungsten.

10. The test-object according to any one of claims 1 to 9, **characterized by** the fact that the placing means (13, 14) are constituted by a rod- or tube-type elongated metal member (13), for example made of titanium or aluminum, an end of which is intended to be inserted into a hole (8a) made in the sphere (8) and to be secured thereto for example through screwing, and by a plate (14) which is fixed to the other end of the elongated member (13) and which is arranged to serve as a counterweight to the test-object and to the elongated member (13) when the plate (14) is arranged relative to the radiotherapy treatment apparatus.

11. The test-object according to claim 10, **characterized by** the fact that the plate (14) comprises means (18) for adjusting the level of said plate (14) relative to the plane of a patient support (2) of the radiotherapy treatment apparatus when placing the test-object.

12. The test-object according to any one of claims 10 or 11, **characterized by** the fact that the sphere (8) comprises a radial channel having a diameter slightly greater than that of the ball (9), said channel being plugged by a rod (8b) made of a material having an electron density d2 able to protrude from the surface of the sphere (8), and by the fact that the elongated member (13) is able to connect to the sphere by screwing at the periphery of the rod (8b), the latter being able to comprise an axial recess having a shape complementary to the shape of the rod (8b) when the latter is protruding, to accommodate this protruding part.

13. The test-object according to any one of claims 10 to 12, **characterized by** the fact that the elongated member (13) is secured to the sphere (8) at the intersection of two visible equatorial lines (10a, 11a, 12a).

14. A method for making a test-object as defined in any one of claims 1 to 13, **characterized by** the fact it comprises the steps of:
- providing a sphere (8) made of a material having an electron density d2 and digging with a half-spherical tip countersink cutter, having a diameter equal to that of the spherical ball (9) made of a material having an electron density d1, a cylindrical channel from a point on the surface of the sphere (8), following a radius thereof, by digging the sphere (8) beyond the center thereof over a distance equal to half the diameter of the ball (9) made of a material having an electron density d1;
- inserting in this channel until the bottom said ball (9);
- plugging the remaining hole with a rod (8b) made of a material having an electron density d2 having a diameter equal to that of said ball (9), at the end of which the reverse shape of the half ball (9) was dug, the rod (8b) being therefore adhered in the channel with an adhesive material having an electron density d2, and the rod (8b) being able to protrude from the surface of the sphere (8) ;
- forming an internally-threaded, greater diameter part in the vicinity of the surface of the sphere (8), at the base of the protruding part of the rod (8b) when the latter is protruding;
- screwing an elongated member (13) carrying at one end a male thread (13a) to the internal thread (8a) of the sphere (8), the elongated member (13) optionally comprising an axial recess, for accommodating the protruding part of the rod (8b) when the latter is protruding;
- securing the other end of the elongated member (13) to a plate (14);
- optionally forming the strip-shaped equatorial areas (10, 11, 12) either by dull rubbing with a 3D machine-tool, or by painting in a dark color, able to scatter or absorb wavelengths in the visible spectrum, advantageously such that the elongated member (13) is secured to the sphere at the center of the overlapping region between two strip-shaped equatorial areas (10, 11, 12);
- forming the visible lines, and if applicable the additional visible lines (10a, 11a, 12a), by etching or printing onto the surface of the sphere (8) with a 3D machine-tool, advantageously such that the elongated member (13) is secured to the sphere at the point of convergence of two visible equatorial lines; and
- optionally coloring the visible lines, and if applicable the additional visible lines (10a, 11a, 12a), by hand-painting with a paintbrush.

15. A method for checking the coincidence, the orthogonality and the position in the space of a treatment room of the locating means (5, 6) of the isocenter of a radiotherapy treatment apparatus, said isocenter being the intersection point of the three theoretical rotation axes (V', H', C') of the radiotherapy treatment apparatus, **characterized by** the fact that it comprises:
- activating the locating means (5, 6) of the three theoretical axes (V', H', C') to visually represent the three theoretical axes (V', H', C');
- placing a test-object as defined in any one of claims 1 to 13 at the point of convergence or observed isocenter (G') of the three theoretical axes (V', H', C');
- visually observing if each of the locating means (5, 6) of the three theoretical axes (V', H', C') follows the respective corresponding alignment-positioning means (10a, 11a, 12a) on the test-object;
- according to the one or more observed differences, modifying the adjustment of the locating means (5, 6) so that the locating means (5, 6) of the theoretical axes (V', H', C') follow the respective corresponding alignment-positioning means (10a, 11a, 12a) on the test-object, in order to ensure the coincidence, the orthogonality and the spatial position of the locating means (5, 6) of the three theoretical axes (V', H', C').

16. A method for searching for the isocenter of a radiotherapy treatment apparatus, using a test-object as defined in any one of claims 1 to 13, outer means and/or means carried by the apparatus being provided for visually representing the three theoretical rotation axes (V', H', C') of the apparatus and their intersection point or isocenter, the method being **characterized by** the fact that it comprises:
- checking the coincidence, the orthogonality and the spatial position of the locating means (5, 6) of the three theoretical axes (V', H', C') in accordance with the method defined in claim 15;
- with the test-object staying at the theoretical isocenter (G'), irradiating the test-object with a radiation beam emitted from the collimator (3), said beam being sensed by the radiation sensing means (4) of the treatment apparatus, and the irradiation being performed at different positions of the patient support (2), of the stand (1) and of the collimator (3) around each of their rotation axes (V, H, C);
- analyzing the obtained images;
- determining the actual position of the three rotation axes (V, H, C) and of their point of convergence (G), actual isocenter of the radiotherapy treatment apparatus;
- adjusting the locating means (5, 6) of the three theoretical axes (V', H', C') so that the actual (G) and theoretical (G') isocenters match.
